Europäisches Patentamt

European Patent Office

Office européen des brevets

(11) Numéro de publication: **0 420 759 A1**

## (12) DEMANDE DE BREVET EUROPEEN

(21) Numéro de dépôt: 90402684.6

(22) Date de dépôt: 28.09.90

(51) Int. Cl.5: **C12N 15/86**, C12N 15/45, C12N 15/48, A61K 39/12, A61K 39/17, A61K 39/21

(30) Priorité: 29.09.89 FR 8912764
15.06.90 FR 9007506

(43) Date de publication de la demande:
03.04.91 Bulletin 91/14

(84) Etats contractants désignés:
AT BE CH DE DK ES FR GB GR IT LI LU NL SE

(71) Demandeur: INSTITUT NATIONAL DE LA
RECHERCHE AGRONOMIQUE
145, rue de l'Université
F-75341 Paris Cédex 07(FR)

(72) Inventeur: Nigon, Victor-Marc
9 rue Condorcet
F-69100 Villeurbanne(FR)
Inventeur: Verdier, Gérard
5-bis rue de la Libération
F-69270 Fontaines S/Saone(FR)
Inventeur: Chebloune, Yahia
5 allée des Cèèdres
F-69100 Villeurbanne(FR)

Inventeur: Ronfort, Corinne
8 rue des Granges
F-69890 La Tour de Salvagny(FR)
Inventeur: Bouquet, Jean-François
Chemin des Charmilles
F-69280 St. Consorce(FR)
Inventeur: Cosset, François Loic
2 Montée Allouche
F-69001 Lyon(FR)
Inventeur: Drynda, Antoine
9 impasse du Presbytère
F-69008 Lyon(FR)
Inventeur: Rey-Senelonge, Arielle
35 allée de la Scarpe
F-69140 Rillieux La Pape(FR)
Inventeur: Legras, Catherine
13 Chemin du Mont-Pilat
F-69120 Vaulx-en-Velin(FR)

(74) Mandataire: Ahner, Francis et al
CABINET REGIMBEAU, 26, avenue Kléber
F-75116 Paris(FR)

(54) **Procédé de préparation de composition immunisante.**

(57) La présente invention concerne un procédé de préparation d'une composition immunisante constituée par l'association d'une protéine immunogène et de fragments membranaires de cellules, ladite protéine étant produite par lesdites cellules présentée sous forme intégrée à leurs membranes, caractérisé en ce que ladite protéine immunogène est produite par l'expression dans des cellules hôtes appropriées d'un vecteur d'intégration et d'expression d'un gène étranger codant pour ladite protéine dans lesdites cellules hôtes.

EP 0 420 759 A1

## PROCEDE DE PREPARATION DE COMPOSITION IMMUNISANTE

La présente invention concerne un procédé de préparation d'une composition immunisante utilisant des vecteurs d'intégration et d'expression d'un gène étranger dans des cellules hôtes.

Selon l'invention, une protéine immunogène fabriquée sur cellules et présentée sur fragments membranaires desdites cellules est dotée de capacité immunogène considérablement améliorée par rapport à ladite protéine purifiée.

Plus précisément, la présente invention concerne un procédé de préparation d'une composition immunisante constituée de l'association d'une protéine immunogène et de fragments membranaires de cellules, ladite protéine étant produite par lesdites cellules en se présentant sous forme intégrée à leurs membranes.

Selon une caractéristique tout à fait avantageuse de la présente invention, la protéine immunogène est produite par l'expression dans des cellules hôtes appropriées d'un vecteur d'intégration et d'expression d'un gène codant pour ladite protéine dans lesdites cellules hôtes.

Plus particulièrement, ledit vecteur d'intégration et d'expression sera un vecteur rétroviral défectif comportant un gène de sélection et le gène de ladite protéine immunogène, ce vecteur étant construit de façon à optimiser la production de la protéine et de sorte qu'elle s'intègre à la membrane des cellules dans laquelle elle est produite.

Les compositions selon l'invention, comportent avantageusement un adjuvant, notamment de l'adjuvant complet de Freund.

La présente invention a également pour objet un procédé de préparation d'une composition, caractérisé en ce que il comporte les étapes suivantes :

a) la protéine immunogène est produite par l'expression dans des cellules hôtes appropriées d'un vecteur d'intégration et d'expression d'un gène étranger codant pour ladite protéine dans lesdites cellules hôtes, le vecteur étant construit de façon à optimiser la production de ladite protéine et de sorte qu'elle s'intègre à la membrane des cellules dans laquelle elle est produite,

b) les cellules hôtes sont soumises à sélection notamment par l'antibiotique correspondant au gène de sélection, et les cellules ainsi sélectionnées sont multipliées,

c) on traite les cellules obtenues de façon à inactiver les acides nucléiques cellulaires et viraux qu'elles contiennent,

d) on récupère et purifie au besoin les constituants membranaires associés à la protéine immunogène.

Dans un mode particulier de réalisation d'un procédé de préparation de composition immunisante selon l'invention, le procédé comporte les étapes suivantes :

- production d'un vecteur rétroviral défectif présenté sous forme d'ADN plasmidien comportant un gène de sélection et le gène d'une protéine immunogène, le vecteur étant construit de façon à optimiser la production de la protéine immunisante, la structure de cette protéine et du vecteur d'intégration étant construits de telle sorte que la protéine s'intègre à la membrane de la cellule dans laquelle cette protéine est produite ;

- transfection de ce vecteur sur une culture de cellules assistantes ("helper") d'où il résulte la production d'une préparation virale ("helper-free") de ce même vecteur sous forme de virus défectif doué de capacité infectieuse ;

- la préparation virale obtenue est employée pour infecter une culture de cellules ordinaires de sorte qu'aucun virus transmissible ne peut se former ;

- les cellules sont ensuite soumises à sélection par l'antibiotique correspondant au gène de sélection employé selon la procédure connue de l'homme de l'art ;

- les cellules ainsi sélectionnées sont multipliées selon une procédure classique ;

- on traite les cellules obtenues de façon à inactiver les acides nucléiques cellulaires et viraux qu'elles contiennent notamment par irradiation UV puis congélation-décongélation et on récupère les constituants membranaires associant la protéine immunogène, constituants que l'on purifie au besoin.

Ce mode de préparation s'est avéré particulièrement performant et n'a jusqu'à présent jamais été employé dans la préparation d'un vaccin.

Le brevet US 4 323 555 décrit un procédé de préparation de composition vaccinante contre le virus BLV comportant des fragments membranaires de cellules. Toutefois, ces compositions sont obtenues à partir de cellules tumorales infectées par le virus BLV entier. Plus précisément, ces cellules sont issues de lymphosarcomes ovins et bovins provoqués par le virus BLV et ne peuvent être utilisés que pour une vaccination contre le BLV. Ce procédé ne permet pas de préparer une composition comportant une protéine immunologique spécifique déterminée associée à des fragments membranaires de cellules.

Comme c'est le cas dans le procédé selon l'invention qui est applicable à la préparation de diverses compositions vaccinantes variées en fonction du gène utilisé comme antigène introduit dans les vecteurs et codant pour une protéine donnée. Le procédé selon l'invention permet en effet de choisir d'immuniser à l'aide d'une protéine particulière à partir de son gène de structure que l'on insère dans les vecteurs. On peut aussi selon l'invention faire exprimer dans ces mêmes cellules plusieurs antigènes pour obtenir des compositions immunisantes contre diverses infections virales.

En outre, le procédé selon l'invention ne met pas en oeuvre des cellules tumorales, et permet un contrôle de ses divers paramètres. En effet, dans le procédé selon l'invention, les cellules sont infectées in vitro par des virions recombinants obtenus à partir de vecteurs rétroviraux défectifs pour leur multiplication. L'étape de production des virions recombinants est contrôlée. Les virions recombinants sont produits sur des cellules assistantes ("helper"). Du fait de leur défectivité, ces virions ne se multiplient pas sur les cellules utilisées ultérieurement comme matériel vaccinant. En outre, les constructions vectorielles selon l'invention permettent, comme on l'a vu, d'optimiser la production d'une protéine immunologique spécifique. Enfin, le procédé selon l'invention, permet la sélection des cellules immunisantes et exprimant l'antigène dont le gène est porté par les vecteurs. Cette sélection est réalisée in vitro à partir de gènes de résistances à des antibiotiques portés par les vecteurs simultanément au(x) gène(s) d'intérêt pour la vaccination.

Dans un mode de réalisation particulier du procédé selon l'invention, on utilise des vecteurs aviaires que l'on cultive sur des cellules aviaires notamment des cellules CEF (fibroblastes d'embryon de poulet) ou des cellules hépatiques LMH de poulets.

Une protéine immunogène fabriquée sur cellules aviaires et présentée sur fragments membranaires desdites cellules aviaires est dotée de capacités immunogènes considérablement améliorées et ce même sur mammifères par rapport à ladite protéine purifiée.

L'emploi d'un vecteur rétroviral aviaire pour préparer une telle protéine permet de maîtriser de façon parfaite le processus d'intégration du gène de la protéine immunogène et le processus de production de ladite protéine dans des cellules aviaires. On peut ainsi optimiser tous les éléments tels que l'activité de transcription, la production de la protéine et la présentation de cette protéine sur des récepteurs capables de multiplier son activité immunogène. Tous ces résultats sont plus difficiles à obtenir par une transfection banale.

Bien sûr l'emploi d'un vecteur aviaire nécessite la culture de cellules aviaires. Toutefois, le même résultat peut être obtenu avec des vecteurs et cellules d'une autre origine par exemple un vecteur de mammifère cultivé sur des cellules hôtes de mammifère tel qu'un vecteur murin.

Des vecteurs viraux d'intégration et d'expression d'un gène hétérologue dans des cellules aviaires ont été décrits dans les demandes de brevets européen et international EP 178 996 et PCT/FR 88 00487 (WO 89/03877). Il conviendra de s'y reporter pour la meilleure intelligence de la présente demande de brevet et plus particulièrement pour la description détaillée de vecteurs viraux aviaires utiles selon la présente invention notamment les vecteurs PTXN3′ et PTXN5′.

Dans un mode de réalisation particulier de l'invention on utilisera comme vecteur d'intégration et d'expression du gène de la protéine immunogène dans des cellules aviaires, un vecteur constitué par tout ou partie du génome proviral de l'érythroblastose aviaire ou d'un virus apparenté dans lequel le gène de la protéine immunogène et le gène de sélection remplacent les gènes v-erbA et v-erbB et en ce que lesdits gènes se trouvent soit sous le contrôle d'un promoteur de LTR du même virus, auquel cas lesdits gènes miment les gènes qu'ils remplacent, soit sous le contrôle d'un promoteur hétérologue auquel cas une séquence att additionnelle est positionnée en amont dudit gène promoteur hétérologue.

Les virus aviaires plus particulièrement concernés par la présente invention sont l'AEV et les virus apparentés du type ALSV, ainsi que les virus non défectifs du type RAV.

Dans un mode de réalisation utile notamment pour l'intégration dans des cellules de poulets CEF ou des cellules hépatiques LMH, le gène de la protéine immunogène se trouve sous la dépendance de LTR aviaire et leur mécanisme de traduction mime celui des gènes qu'ils remplacent.

De façon appropriée, le vecteur comporte le gène de sélection en position v-erbA, et le(s) gène(s) de la protéine immunogène en position v-erbB.

Avantageusement, selon une autre caractéristique de ces vecteurs, le gène de sélection et le gène de la protéine immunogène se trouvent traduits à partir de l'AUG du gène gag ou à partir de leurs propres AUG, mais toujours dans le même cadre de lecture que celui du gène gag. Ainsi, le cas échéant, le gène de sélection est traduit à partir de l'AUG du gène gag et le gène de la protéine immunogène est traduit à partir de l'AUG du gène gag ou son propre AUG.

Avantageusement, un codon stop est introduit entre le gène gag et le gène de la protéine immunogène ou l'AUG propre dudit gène de la protéine immunogène.

De préférence, le codon stop est situé à une distance de 60 à 70 nucléotides du codon AUG dudit gène de la protéine immunogène, notamment 65.

Dans un autre mode de réalisation de l'invention, le vecteur d'intégration et d'expression est tel que le gène de sélection est en position v-erbA sous la dépendance d'un promoteur LTR5' aviaire et le gène de la protéine immunogène est en position v-erbB sous la dépendance d'un promoteur hétérologue, le vecteur comportant alors une séquence att supplémentaire positionné en amont du promoteur hétérologue.

Dans les vecteurs qui ont une séquence att supplémentaire, on peut déléter une partie de la séquence U5 de la LTR5' de telle sorte qu'après un cycle rétroviral ne reste fonctionnelle que la séquence att supplémentaire qui assure l'intégration.

De préférence, on effectue une délétion de 23 pb dans la région 3' terminal de la séquence U5 de la LTR5'.

Avantageusement, le vecteur est caractérisé en ce que le gène de la protéine immunogène est en position v-erbB encadré par le promoteur hétérologue et la séquence de polyadénylation hétérologue, et en ce que l'ensemble promoteur - gène de la protéine immunogène - séquence de polyadénylation est dans la même orientation ou en orientation opposée au sens transcriptionnel rétroviral.

On peut citer notamment comme promoteur hétérologue le promoteur du virus simien SV40.

La mise en oeuvre du procédé selon l'invention nécessite également l'utilisation de lignées permanentes de cellules assistantes ("helper") capables de produire des préparations virales "helper-free". Les lignées assistantes fournissent au génome défectif, les protéines gag-pol-env nécessaires pour lui permettent de former des virions et permettent donc d'utiliser des vecteurs viraux défectifs sans adjonction de virus assistants.

Selon la présente invention, on pourra utiliser notamment comme vecteur capable de transformer une cellule aviaire normale en cellule "helper" un vecteur comportant tout ou partie des trois gènes gag-pol-env du virus RAV-1 ou du virus RAV-2 placés sous le contrôle transcriptionnel d'une LTR du même virus dans le génome duquel diverses délétions ont été apportées afin de supprimer l'aptitude à l'encapsidation de l'ARN produit par ce vecteur.

Dans un mode de réalisation, on utilise comme cellules de départ pour préparer les cellules assistantes, des cellules de la lignée permanente de cailles QT6.

Dans un mode de réalisation, notamment lorsque le vecteur d'intégration et d'expression comprend comme gène de protéine immunisante le gène env, on a pu supprimer dans le vecteur visant à transformer les cellules normales en cellules "helper" le gène env. Dans d'autres modes de réalisation, les gènes gag et pol peuvent être délétés et remplacés par un gène de sélection, le gène env subsistant étant précédé par son site accepteur d'épissage.

Un exemple de réalisation de l'invention est une composition d'immunisation contre le virus RSV, caractérisée en ce que ladite protéine est la protéine env associée à des fragments membranaires de cellules CEF.

Dans un autre exemple, la présente invention a pour objet une composition immunisante contre la maladie de Newcastle. Plus précisément, dans un mode réalisation de l'invention, la protéine est l'hémaglutinine-neuraminidase (HN) du virus de la maladie de Newcastle.

Comme mentionné précédemment, on trouvera décrits d'autres caractéristiques des vecteurs aviaires utiles dans le procédé selon l'invention dans la demande de brevet international WO 89/03877.

D'autres caractéristiques et avantages de la présente invention apparaîtront à la lumière de la description détaillée d'un mode de réalisation qui va suivre.

L'exemple 2 est illustré par les figures 1 à 5.

La figure 1 représente la construction du vecteur SK$^+$Hn-

Les figures 2 à 4 représentent les constructions des vecteurs pHnN$_1$, pHnN$_2$ et pHnN3.

La figure 5 représente la structure des vecteurs des figures 2 à 4 véhiculant le gène Hn du NDV.

La figure 6 représente la carte du plasmide PNDV-71 :

ATG : codon d'initiation de Traduction

TGA : codon terminateur de Traduction

Amp$^R$, Tcl$^R$: gènes de résistances, porté par la séquence pLBR322, aux antibiotiques ampicilline, Tetracycline ;

ori : origine de réplication du plasmide

F, L : résidu des gènes F et L du NDV.

Hn : gène Hn

polyAf, poly AHn : séquences de polyadénylation des messagers des gènes F et Hn.

Les grandes boîtes représentent les LTRs, le gène Hn, et le gène de sélection. Les petites boîtes représentent les résidus des gènes rétroviraux. Les lignes fines représentent les ARN transcrits à partir des vecteurs, tandis que les lignes épaisses représentent les protéines synthétisées. Les triangles blancs ou noirs représentent les codons initiateurs ou terminateurs de traduction. **ds** et **as** sont les sites donneurs et accepteurs d'épissage.

## EXEMPLE 1 : Composition immunisante contre les virus ALSV responsables de leucoses et sarcomes aviaires.

On a employé un vecteur contenant un gène env de rétrovirus aviaire RAV-1 appartenant au sous groupe A. Les animaux (des poulets) ont reçu ce gène ou les produits de son activité sous diverses formes détaillées ci-après. Sur les animaux ainsi traités, on a examiné d'une part la production d'anticorps anti-env (titrés par sero neutralisation d'une préparation virale), et d'autre part, la production de tumeurs après épreuve résultant de l'injection d'une préparation de virus RSV.A (Rous Sarcoma Virus de sous groupe A) (3,6 $10^2$ PFU/animal), 35 jours après le premier traitement immunisant.

### 1. Construction des vecteurs

Les gènes env de sous-groupes A et B, d'origines RAV-1 et RAV-2 respectivement, ont été insérés dans le veceur de base PTXN5' (portant déjà le gène néo) préalablement délété de la séquence J (contenant le site accepteur d'épissage de l'AEV) et du résidu du gène env de l'AEV. Ces vecteurs rétroviraux appelés pNEA et pNEB ont été décrits dans le brevet WO 89/03877 à l'exemple 2-b.4).

Le vecteur non encapsidable pGPH servant à transformer des cellules aviaires normales en cellules assistantes, a été produit à partir de pHF13 dans lequel a été inséré le gène hygro conférant résistance à l'hygromycine B à la place du gène env (en aval du site accepteur d'épissage). Ce vecteur pGPH portant les gènes gag et pol du RAV-1 a été décrit dans le brevet WO 89/03877 exemple 4 : 5.1.4.

### 2. Obtention des lignées transcomplémentantes HAYDEE et ISOLDE

Des cellules de caille QT6 ont été transfectées par l'ADN du vecteur pGPH, et sélectionnées par l'hygromycine. 23 colonies hygro$^+$ ont été isolées, et testées pour leur production de p27$^{gag}$ extra et intracellulaire. La moitié de ces clones produisaient une quantité de p27$^{gag}$ équivalente à celle produite par des cellules QT6 infectées par RAV-1.

9 de ces lignées ont été transfectées par l'ADN du vecteur pNEA, et sélectionnées par la néomycine. Les surnageants de ces cultures ont été titrés pour la production de particules conférant la résistance à la néomycine (en RFU/ml). Parmi ces cultures, 2 produisaient des particules à un taux significatif dans des conditions "helper-frée", dont un à très haut titre (plus de $10^5$ RFU/ml). On a appelé HAYDEE le clone hygro$^+$ qui peut produire cette dernière culture. Ce clone est donc transcomplémentant pour l'expression d'un vecteur défectif portant un gène env.

La lignée HAYDEE a été transfectée par l'ADN du vecteur non encapsidable pPhEA qui est décrit dans la demande de brevet WO 89/03877 à l'exemple 4 : 5.1.6. Ce vecteur porte le gène de résistance à la phléomycine (gène phléo), ainsi que le gène env de sous-groupe A. Après sélection par la phléomycine, des clones phléo$^+$ ont été isolés. Sur 11 d'entre eux, la production du gène env a été testée par un test d'interférence, grâce à un stock viral superinfectant de sous-groupe A. En effet, la protéine codée par le gène env sature les récepteurs superficiels spécifiques de sous-groupe et empêche une nouvelle infection par un rétrovirus du même sous-groupe. Parmi ces clones, 2 se sont avérés être aussi interférant que des cellules de caille QT6 infectées par le virus RAV- 1. 4 de ces clones ont été transfectés par l'ADN du vecteur rétroviral défectif pNL53 décrit dans la demande de brevet WO 89/03877 à l'exemple 2-2). Les surnageants de ces cultures ont été titrés pour leur capacité à transmettre, à des cellules cibles, le vecteur PNL53 dans des conditions "helper-free". Le clone le plus producteur a été dénommé ISOLDE ; il peut produire des vecteurs rétroviraux entièrement défectifs, à un titre de l'ordre de $10^4$ à $10^5$ RFU/ml.

### 3. Propriétés du virus NEA

Des cellules de caille QT6 ont été infectées par le vecteur rétroviral PNEA produit par la lignée HAYDEE transfectée par le pNEA. Des clones de QT6 résultant de cette infection ont été isolés (après sélection par la néomycine). Sur ces clones a été testée la production du gène env par test d'interférence avec un stock viral interférent pseudotypé en sous-groupe A.

Il s'est avéré que 80 % de ces clones exprimait le gène env, attestant la capacité du vecteur NEA à transférer l'expression du gène env à des cellules en cultures.

## 4. Préparation du matériel immunisant

### 4.1. Virus défectif NEA (modalité a) dans le tableau II

Le virus NEA produit par la lignée transcomplémentante HAYDEE transfectée par le vecteur pNEA, a été concentré 100 fois par ultracentrifugation (30000 RPM, 4°C, 20 min). Les stocks viraux sont conservés à - 80°C.

### 4.2. Surnageant de la lignée ISOLDE (modalité b) dans le tableau II

Ce surnageant, produisant les protéines rétrovirales à l'exclusion de matériel génétique rétroviral, a été concentré 100 fois (mêmes conditions que pour le virus NEA).

### 4.3. Préparations faites à partir de CEFs infectées par le vecteur NEA (modalité c) dans le tableau II

Des fibroblastes embryonnaires de poulet (CEF) sont infectés par la préparation virale "helper-free" du vecteur NEA, puis sélectionnés par le G418. Les cellules neo[+] sont alors amplifiées, et on teste dans leur surnageant l'absence de virus sauvage. Ces cellules sont irradiées par les UV (à 254 nm) pendant 5 min, trypsinées puis resuspendues dans du PBS à la concentration de $3 \times 10^6$ cellules par ml. Les préparations sont alors congelées à - 20°C, et décongelées juste avant injection chez l'animal.

### 4.4. Préparations faites à partir de CEFs infectés par le virus RAV- 1 (modalité d) dans le tableau II

Des CEF sont infectés par un surnageant du virus RAV-1, et amplifiées. Avant de les récolter selon les mêmes modalités que celles décrites plus haut (en 4.3.), on contrôle la présence du virus RAV-1.

## 5. Injections des animaux

Les animaux qui à ce jour ont donné les résultats les plus complets étaient âgés de 3 mois au début du traitement, et répartis en lots de 9 à 11 animaux traités de façon homogène (voir tableau II)

### 5.1. Protocole d'immunisation

Les animaux ont reçu une injection par voie intraveineuse selon les modalités a) (environ $10^7$ RFU/animal), b) (1 ml par animal), c) ($3 \times 10^6$ cellules recombinantes par animal), d) ($3 \times 10^6$ cellules virémiques par animal), e) ($3 \times 10^6$ cellules témoins par animal).

Un rappel d'injection est effectué 15 jours après la primo-injection, selon les mêmes modalités.

Un prélèvement de sang est effectué tous les 8 jours, et est destiné à la préparation de sérum pour tester la présence d'anticorps.

5 semaines après la primo-injection, tous les animaux (sauf ceux traités selon la modalité d) sont injectés par voie sous-cutanée par $3,6 \times 10^2$ PFU/animal d'une préparation virale de RSV de sous-groupe A.

### 5.2. Résultats

## 1) Contrôle des anticorps neutralisants

Principe :

Les anticorps neutralisants abolissent spécifiquement l'activité infectieuse du rétrovirus contre lequel ils sont dirigés. Pour contrôler les sérums des animaux traités, nous avons employé un stock viral NL/RAV 1 (véhiculant les gènes $Néo^R$ et Lac Z) incubé avec des dilutions des sérums à tester (1, 1/2, 1/4... 1/8192), qui a servi ensuite pour infecter des cellules QT6 sur lequelles la détection de l'activité B-galactosidase a été effectuée par coloration au X-Gal.

Les résultats de séroneutralisation obtenus avec les diverses dilutions de sérums ont permis de définir pour chaque sérum la dilution permettant de neutraliser 50 % et 100 des virus NL : $ND_{50}$ et $ND_{100}$.

### TABLEAU I

| (1) | (2) | | | | | | | | (3) | (4) |
|-----|-----|-----|-----|-----|-----|-----|-----|-----|-----|-----|
|     | (a) | (b) | (c) | (d) | (e) | (f) | (g) | (h) |     |     |
| 1 | 0 | 0 | 0 | 7 | 21 | 47 | 85 | 129 | 6000 | <128 |
| 2 | 0 | 2 | 21 | 45 | 83 | 116 | 144 | 172 | 2000 | <128 |
| 3 | 0 | 3 | 11 | 33 | 57 | 95 | 106 | 123 | 6000 | < 64 |
| 4 | 8 | 30 | 47 | 83 | 155 | 216 | NT | NT | 640 | < 4 |
| 5 | 0 | 0 | 0 | 3 | 5 | 13 | 26 | 58 | >8000 | <256 |
| 6 | 0 | 0 | 1 | 4 | 9 | 33 | 101 | 211 | 4800 | < 64 |
| 7 | 0 | 0 | 11 | 18 | 27 | 56 | 84 | 148 | 6000 | < 32 |
| 8 | 0 | 0 | 0 | 0 | 2 | 10 | 36 | 64 | >8000 | <128 |
| 9 | 0 | 3 | 11 | 23 | 54 | 83 | 118 | 147 | 4000 | < 32 |
| NP | 300 | 300 | NT | NT | NT | NT | NT | NT | - | - |

**(1)** = sérum testés ; **(2)** = nombre de cellules B-gal pour chaque dilution des sérums : (a) = 1/16 ; (b) = 1/64 ; (c) = 1/256 ; (d) = 1/512 ; (e) = 1024 ; (f) = 1/2048 ; (g) = 1/4096 ; ( ) = 1/8192 ; **(3)** = ND50 ; **(4)** = DN100 ; NP = Non Protégé.

### TABLEAU I :

Résultats de séroneutralisation représentés par le nombre de cellules bleues pour chacune des dilutions du sérum employé. La dernière dilution neutralisant 100 % le virus NL ($ND_{100}$) et la dilution permettant de neutraliser 50 % du virus : $ND_{50}$ sont représentées dans les deux dernières colonnes. Les valeurs de $ND_{50}$ rapportés dans l'avant dernière colonne sont déterminées à partir des courbes rélisées avec les valeurs de neutralisation obtenues pour les dilutions de chaque sérum et rapportées dans les colonnes 2 à 9. Les sérums des 9 animaux étudiés correspondent à la modalité c- (voir Tableau II).

## 2) Le bilan d'une expérience est rapporté dans le Tableau II ci-après

7

EP 0 420 759 A1

TABLEAU II

| (1) | (2) | (3) | (4) | (5) | (6) | (7) |
|---|---|---|---|---|---|---|
| a)NEA HF | 9 | 0 | 9/9 | 0 % | 100 % | 0 % |
| b)ISOLDE | 0 | 2/9 | 7/9 | 22 % | 78 % | 33 % |
| c)CEF NEA | 9 | 9/9 | 0/9 | 100 % | 0 % | - |
| d)CEF RAVI | 9 | 9/9 | non testé | 100 % | non testé | non testé |
| CEF témoins | 10 | 0/10 | 9/9 | 0 % | 100 % | 0 % |
| non injecté | 11 | 0/11 | 11/11 | 0 % | 100 % | 9 % |

(1) = modalité d'injection ; (2) = effectif des animaux ; (3) = nombre d'animaux produisant des anticorps neutralisants ; (4) = nombre d'animaux porteurs de tumeurs ; (5) = % d'animaux producteurs d'anticorps ; (6) = % d'animaux porteurs de tumeurs ; (7) = % d'animaux régresseurs de tumeurs.

On remarque en particulier qu'aucun animal traité selon la modalité c- n'a développé de tumeurs à la suite de l'injection de RSV.

Une série d'autres expériences visant à étudier divers paramètres pouvant influencer la réponse immune, tels que la prépartion des antigènes, la voie d'immunisation, les doses d'antigène... a eté réalisée se limitant à l'analyse de la production des anticorps neutralisants dans le sérum des animaux. Ces expériences ont été réalisées chez des animaux adultes, d'une part et chez des poussins de 2 jours d'autre part.

1) Chez les animaux adultes

a - Influence du mode de préparation des antigènes

Des CEF/NEA sont irradiés puis récoltés soit après action de la trypsine (modalité a), soit par grattage mécanique de la couche unicellulaire et homogénéisation de la suspension cellulaire (modalité b), soit enfin par grattage et homogénéisation suivis de sonication (10 périodes de 5s à une puissance de 40 W) de la suspension cellulaire (modalité c). Ces préparations cellulaires sont congelées et décongelées avant leur utilisation.

Des poulets âgés de 2 mois (5 par modalité) sont inoculés une seule fois par voie intraveineuse par $10^7$ cellules par animal traitées selon les modalités a, b et c. La $ND_{50}$, pour chaque sérum et chaque semaine pendant 5 semaines post inoculation, a été déterminée (tableau III).

**TABLEAU III :**

Etude de l'effet de la préparation de l'antigène selon les modalités ci-dessus indiquées sur l'efficacité de l'induction de la réponse immune. Les valeurs représentent les dilutions permettant de neutraliser 50 % du virus NL.

8

TABLEAU III

| (1) | (2) | (3) | | | | |
|---|---|---|---|---|---|---|
| | | 7j. | 14j. | 21j. | 28j. | 35j. |
| 1 | Trypsine | 0 | 0 | 0 | 1/4 | 1/4 |
| 2 | (a) | 0 | 0 | 0 | 1/4 | 1/8 |
| 3 | | 0 | 0 | 0 | 1/8 | 1/32 |
| 4 | | 0 | 0 | 0 | 1/4 | 1/8 |
| 5 | | 0 | 0 | 0 | 1/4 | 1/8 |
| 6 | Grattage | 0 | 1/128 | 1/512 | 1/256 | 1/128 |
| 7 | (b) | 0 | 1/128 | 1/512 | 1/512 | 1/128 |
| 8 | | 0 | 1/128 | 1/1024 | 1/512 | 1/256 |
| 9 | | 0 | 1/128 | 1/1024 | 1/2048 | 1/256 |
| 10 | | 0 | 1/128 | 1/512 | 1/256 | 1/64 |
| 11 | Grattage | 0 | 1/128 | 1/2048 | 1/4096 | 1/1024 |
| 12 | sonication | 0 | 1/128 | 1/2048 | 1/1024 | 1/128 |
| 13 | (c) | 0 | 1/128 | 1/1024 | 1/512 | 1/128 |
| 14 | | 0 | 1/128 | 1/128 | 1/512 | 1/1024 |
| 15 | | 0 | 1/128 | 1/2048 | 1/1024 | 1/512 |

(1) = Sérum ; (2) = Modalité de préparation de l'antigène ; (3) = Résultats de Seroneutralisation ($ND_{50}$) en fonction du temps.

On remarquera que les plus fortes valeurs de $ND_{50}$ correspondant aux réponses immunes les plus fortes sont obtenues avec l'antigène préparé selon la modalité (c). La préparation (b) induit une réponse immune assez forte, tandis que la réponse immune la plus faible est observée avec les antigènes traités par la trypsine (a).

b) Influence de la dose d'antigène

Des CEF/NEA préparés selon la modalité (b) ci-dessus, ont été utilisés pour inoculer par voie intramusculaire des poulets âgés de 3 mois. Ces animaux ont été répartis en groupes de 5 et inoculés par des doses différentes d'antigène allant de $3.10^2$ à $3.10^6$ cellules par animal (Tableau IV). 10 jours plus tard, une seconde inoculation dans les mêmes conditions que la première est effectuée. Les sérums sont analysés chaque semaine par séroneutralisation et les valeurs de $ND_{50}$ sont comparées entre les divers groupes (Tableau IV).

**TABLEAU IV :** Effet de la dose d'antigène sur la réponse immune

Dans la 2ème colonne sont rapportés la dose d'antigène inoculée à chaque animal. Dans les 3 dernières colonnes sont rapportés les effectifs des animaux donnant des $ND_{50}$ supérieures ou inférieures à des dilutions choisies.

Les résultats résumés dans le tableau IV démontrent une décroissance de la réponse immune accompagnant la décroissance de la dose d'antigène inoculée. L'inoculation de la faible dose de $3.10^4$ cellules par animal permet de déteminer une faible réponse humorale atteignant une $ND_{50}$ aux dilutions 1/8 à 1/32 35 jours après la première inoculation.

| Effectif des animaux | Dose d'antigène | $ND_{50}$ | | |
|---|---|---|---|---|
| | | 21j. | 28j. | 35j. |
| 5 | $3.10^6$ | $^5> 1/8$ | $^5> 1/32$ | $^5> 1/64$ |
| 5 | $3.10^5$ | $^4> 1\geq 8$ <br> $1 = 1/4$ | $^3> 1\geq 32$ <br> $2< 1/32$ | $^5> 1\geq 64$ |
| 5 | $3.10^4$ | $^3> 1\geq 8$ <br> $2< 1/4$ | $^2> 1\geq 32$ <br> $3< 1/4$ | $^0>$ <br> $1/32$ |
| 5 | $3.10^3$ | $2 = 1/8$ <br> $3< 1/4$ | $5< 1/4$ | non testé |

c) Influence de la voie d'inoculation de l'antigène

Des CEF/NEA préparés selon la modalité (b) (Tableau III) sont inoculés à une trentaine de poulets âgés de 3 mois et répartis en 2 groupes. Au temps T0 les animaux du 1er groupe sont inoculés par la voie intraveineuse à raison de $10^7$ cellules par animal, alors que les animaux du 2ème groupe sont inoculés par la voie intra musculaire. A T10 un rappel est effectué dans les mêmes conditions. Les sérums des animaux sont testés chaque semaine pour leur capacité séroneutralisante et la $ND_{50}$ pour chaque sérum est déterminée. Les effectifs des animaux de chacun des 2 groupes permettant d'obtenir une $ND_{50}$ soit supérieure ou égale à 1/32 soit inférieure à 1/32 sont rapportés dans le tableau V. Ces résultats démontrent une efficacité meilleure de la voie intraveineuse par rapport à la voie intramusculaire à induire une réponse immune. En effet 8 animaux sur 15 du groupe 1 (inoculés par I.V.) permettent d'obtenir une $ND_{50}$ à une dilution supérieure ou égale à 1/32 contre seulement 2/14 dans le second groupe (I.M.). On notera que quelque soit la voie d'immunisation on observe que tous les animaux développent une réponse humorale avec cependant une variabilité au niveau d'une part du temps de latence et d'autre part du niveau d'anticorps produits.

| groupe | effectif | voie d'immunisation | $ND_{50}$ | |
|---|---|---|---|---|
| | | | supérieure à 1/32 | inférieure à 1/32 |
| I | 15 | I.V. | 8 | 7 |
| II | 14 | I.M. | 2 | 12 |

**TABLEAU V :** Comparaison de l'efficacité à induire une réponse immune entre la voie intraveineuse et la voie intramusculaire.

d) Mémorisation de la réponse humorale

A l'issue de l'expérience de l'analyse de l'influence du mode de préparation des antigènes ci-dessus rapportée, les animaux 1, 9, 10; 14 et 15 (tableau III) ont été conservés. A $T_{95j.}$ après la lère immunisation, ces animaux ont été inoculés par $10^7$ cellules de la préparation de CEF/NEA de la modalité (b). Les sérums des animaux ont été récoltés un jour sur deux pendant 2 semaines, et aux jours 21 et 28 après la dernière inoculation, puis testés par séroneutralisation. Les valeurs de $ND_{50}$ pour chaque sérum sont rapportées dans le tableau VI, et sont utilisées pour tracer les courbes de l'évolution de la réponse humorale en fonction du temps et des rappels d'immunisation.

Ces résultats démontrent qu'à la suite de la 1ère et 2ème inoculation de l'antigène la réponse immune se développe progressivement et lentement pour atteindre une production maximale d'anticorps neutralisants 4 à 5 semaines après la 1ère immunisation. Cette réponse décroît pour se stabiliser à une valeur moyenne pendant les 2 mois. Après injection de l'antigène à $T_{95\ j.}$ on observe une production très rapide des anticorps neutralisants. Cette production atteint ou dépasse la valeur de l'activité maximale 2 à 3 fois plus vite. Ces résultats démontrent que cette réponse humorale est mémorisée.

| (1) | (2) | (3) | (4) | (5) | (6) |
|---|---|---|---|---|---|
| 1 | 0 | 0 | 1/4 | 1/4 | 1/4 |
| 9 | 1/128 | 1/1024 | 1/2048 | 1/256 | 1/512 |
| 10 | 1/28 | 1/512 | 1/128 | 1/1024 | 1/128 |
| 14 | 1/128 | 1/128 | 1/512 | 1/1024 | 1/512 |
| 15 | 1/128 | 1/2048 | 1/1024 | 1/512 | 1/512 |

Suite tableau VI :

| (7) | (8) | (9) | (10) | (11) | (12) |
|---|---|---|---|---|---|
| 1/8 | 1/8 | 1/16 | 1/128 | 1/256 | 1/256 |
| 1/512 | 1/512 | 1/2048 | 1/4096 | 1/4096 | 1/2048 |
| 1/256 | 1/256 | 1/1024 | 1/1024 | 1/1024 | 1/256 |
| 1/512 | 1/1024 | 1/8192 | 1/8192 | 1/8192 | 1/4096 |
| 1/512 | 1/512 | 1/1024 | 1/2048 | 1/4096 | 1/4096 |

(1) = animal ; (2) = 14j ; (3) = 21j. ; (4) = 28j. ; (5) = 35j. ; (6) = 3ème inoculation - 95j. ; (7) = 97j. ; (8) = 99 ; (9) = 102 ; (10) = 106 ; (11) = 116 ; (12) = 116 ; (13) = 123.

2) Chez des poussins de 2 jours

Des poussins âgés de 2 jours sont inoculés par voie intrapéritonéale par $10^4$ particules virales du virus NEA en préparation helper-free (condition NEA-HF).

Au temps T3j. et T6j. des rappels dans les mêmes conditions que lors de la première inoculation sont effectués chez tous les animaux.

Au temps T30j., T37j. et T44j. des prélèvements de sang sont effectués chez tous les animaux et les sérums sont récoltés.

Les résultats de l'analyse des sérums par test de séroneutralisation démontrent que tous les sérums présentent une activité neutralisante contre un retrovirus aviaire de sous A.

La valeur de $ND_{50}$ a été déterminée pour tous les sérums du prélèvement T44j.

Sur les 20 poussins étudiés, 12 présentent une valeur de $ND_{50}$ correspondant à une dilution du sérum inférieure ou égale à 1/1024, 4 à une dilution inférieure ou égale à 1/512 et les quatre autres inférieure ou égale à 1/8.

Ces résultats démontrent une efficacité d'infection du virus NEA-HF chez les poussins, puisque la totalité des animaux inoculés à ce stade par la préparation NEA-HF développe une réponse humorale représentée par les anticorps neutralisants. Les cellules infectées au stade poussin se multiplient et détermineraient une réponse immune produisant des anticorps neutralisants à partir de 6-7 semaines chez l'animal, lors de la mise en place du système immunitaire. Cependant cette réponse est d'intensité variable selon les individus.

On notera que ces résultats différents de ceux obtenus chez les animaux adultes puisqu'aucune réponse humorale ni résistance au développement de tumeurs n'avaient été observées chez les animaux traités au stade adulte selon cette modalité (voir modalité (a) Tableau II).

Chez les animaux adultes l'inefficacité d'infection du virus NEA-HF pourrait résulter soit de la destruction rapide des particules virales NEA-HF après inoculation des animaux, soit de la destruction rapide des cellules infectées par le système immunitaire de l'animal.

Les résultats obtenus par inoculation de poussins de 2 jours directement par des virus NEA-HF pourraient conduire à l'utilisation d'une telle modalité y compris sur des embryons de 18 jours afin d'induire une résistance à l'infection par des rétrovirus aviaires leucemogènes ou sarcomatogènes.

**EXEMPLE 2 :** Composition immunisante contre la maladie de Newcastle

## 1. Construction des vecteurs

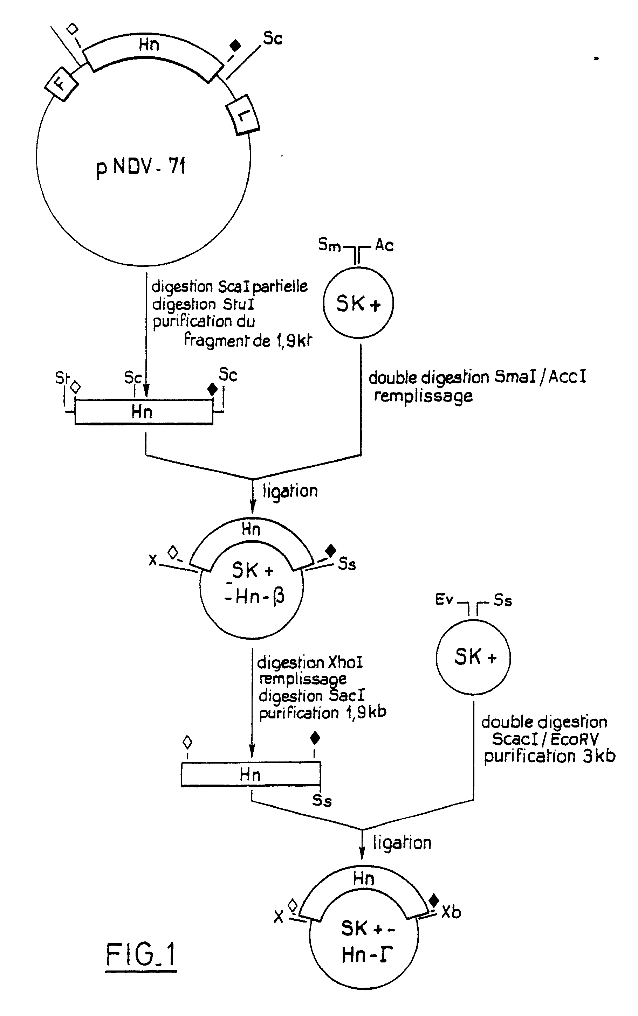

Trois constructions ont été réalisées (vecteurs pNHn2 et PNHn3) à partir des vecteurs de base PTXN3′ et PTXn5′ produits au laboratoire, et du gène Hn codant pour l'hémagglutine-neuraminidase du virus de la maladie de Newcastle (NDV, souche Texas vélogène : Taylor et Journal of virology 1990,64 : 1441-1450) fourni par les établissements Rhône-Mérieux. Le fait que le gène HN provienne de la souche Texas vélogène n'est pas particulièrement important dans la mesure où il s'agit d'un gène modèle. On aurait pû tout aussi bien utiliser des gènes HN provenant d'autres souches. Ces constructions, dont la structure générale et le fonctionnement sont illustrés dans la figure 1, diffèrent entre elles par la position d'insertion du gène Hn dans le vecteur rétroviral de base, ainsi que par les processus conduisant à la traduction de la protéine hémag-glutinine-neuraminidase. Les détails de constructions sont exposés ci-après.

### Construction des plasmides SK⁺-NH-$\beta$ et SK⁺-HNΓ : (voir figure 1).

On isole à partir du plasmide PNDV71 contenant le gène Hn du NDV (souche Texas), un fragment de 1,9 kb contenant ce gène, par double digestion Scal/Stul. Ce fragment est inséré dans les sites remplis Smal et Accl du plasmide SK⁺. Il en résulte le plasmide SK⁺-HN-$\beta$, à partir duquel on isole par digestion Xhol, remplissage, et digestion Sacl un fragment de 1,9 kb contenant le gène Hn. Ce dernier est sous-cloné à nouveau dans le plasmide SK⁺ préalablement digéré par les enzymes ECoRV et Sacl, ce qui conduit à la formation du plasmide SK⁺-HN-Γ.

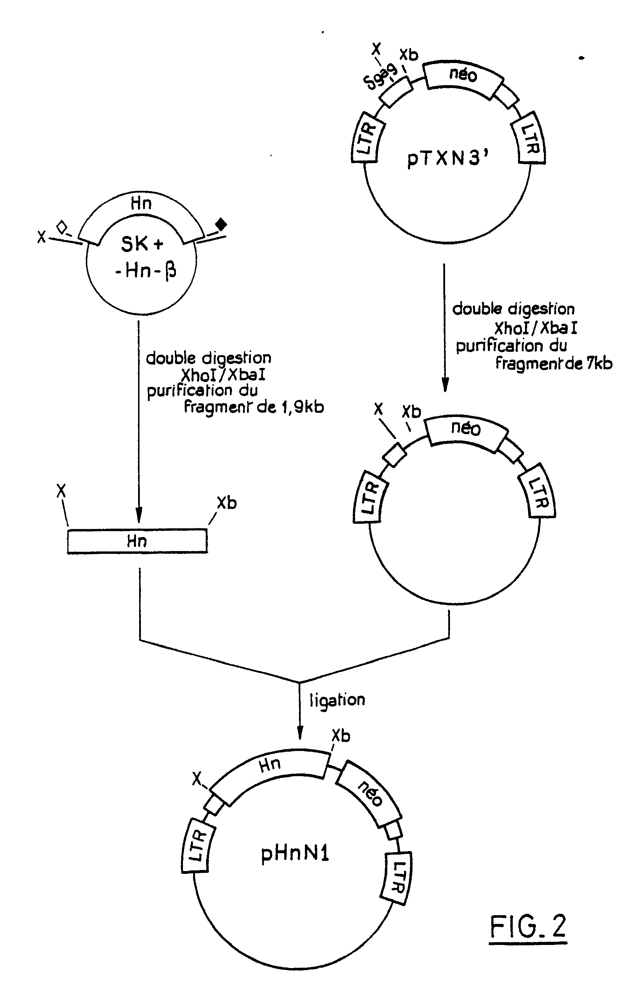

### Construction du vecteur retroviral PHnN1 : (voir figure 2)

On isole par double digestion Xho1/Xba1 à partir du plasmide SK⁺-HN-$\beta$, un fragment de 1,9 kb contenant le gène Hn. Ce fragment est cloné dans les sites Xho1 et Xba1 du vecteur rétroviral PTXN3′ - (contenant le gène néo en position v-erb B). Il en résulte le vecteur pHnN1.

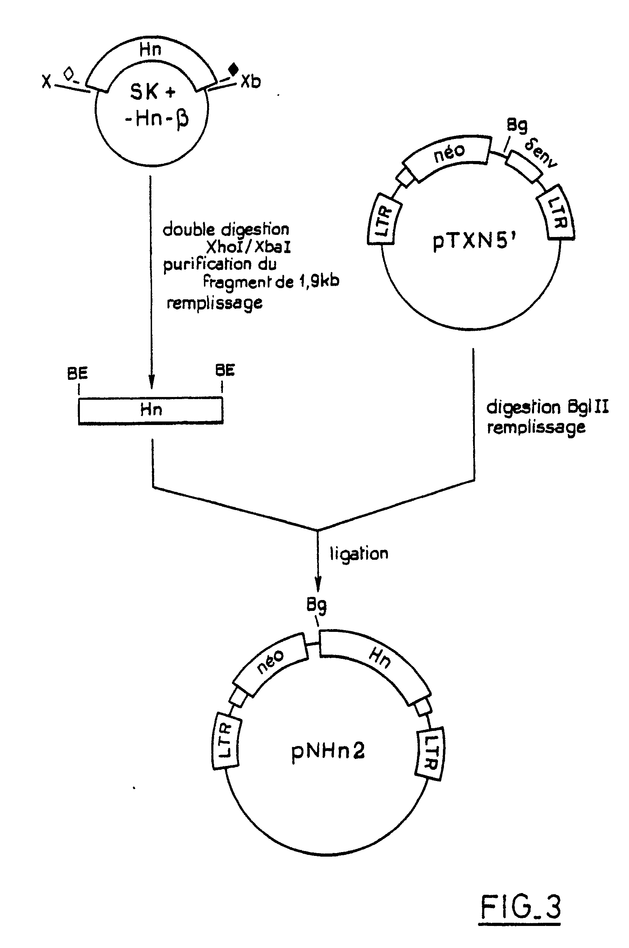

**Construction du vecteur rétroviral pNHn2 :** (voir figure 3)

On isole par double digestion Xho1/Xba1 à partir du plasmide SK$^+$-HN-$\beta$,un fragment de 1,9 kb contenant le gène Hn dont les extrémités sont remplies avant de la cloner dans le site Bg111 (rempli) du vecteur PTXn5$^{'}$. Le vecteur rétroviral résultant est appelé pNHn2.

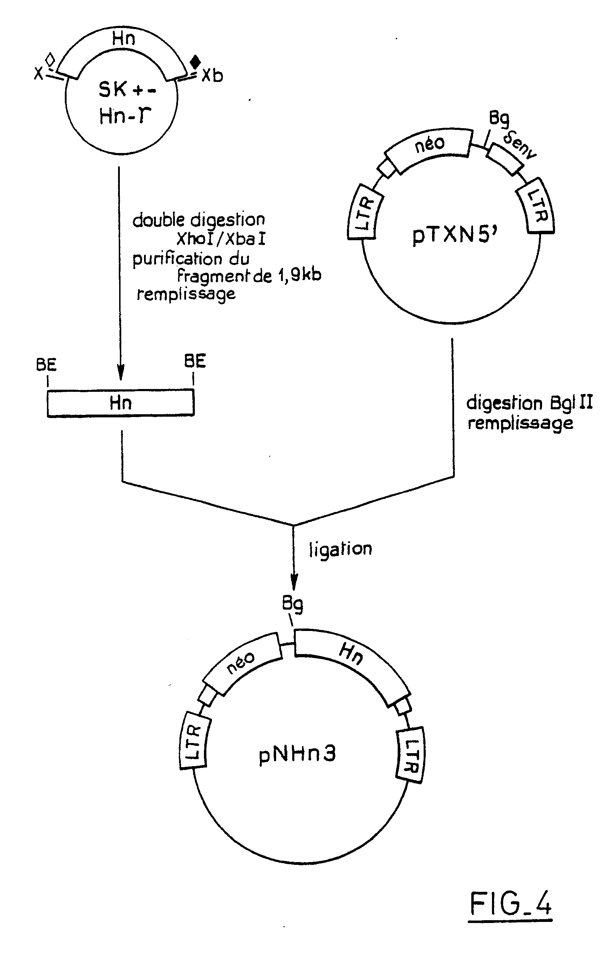

**Construction du vecteur rétroviral pNHn3 :** (voir figure 4)

On isole par double digestion Xho1/Xba1 à partir du plasmide SK$^+$-HNΓ, un fragment de 1,9 kb contenant le gène Hn dont on remplit les extrémités, avant de le cloner dans le site Bg111 (rempli) du vecteur PTXn5$^{'}$. Dans le vecteur rétroviral résultant pNHn3, l'insertion du gène Hn à partir du SK + -HN-Γ conduit à introduire un codon stop, en phase avec le codon initiateur de traduction du gène gag, sur l'ARN sousgénomique. De plus, cette insertion conduit à introduire un "spacer" d'environ 70 nucléotides entre ce codon stop et le codon initiateur du gène Hn, ce qui représente une distance favorable à une ré-initiation optimale de la traduction.

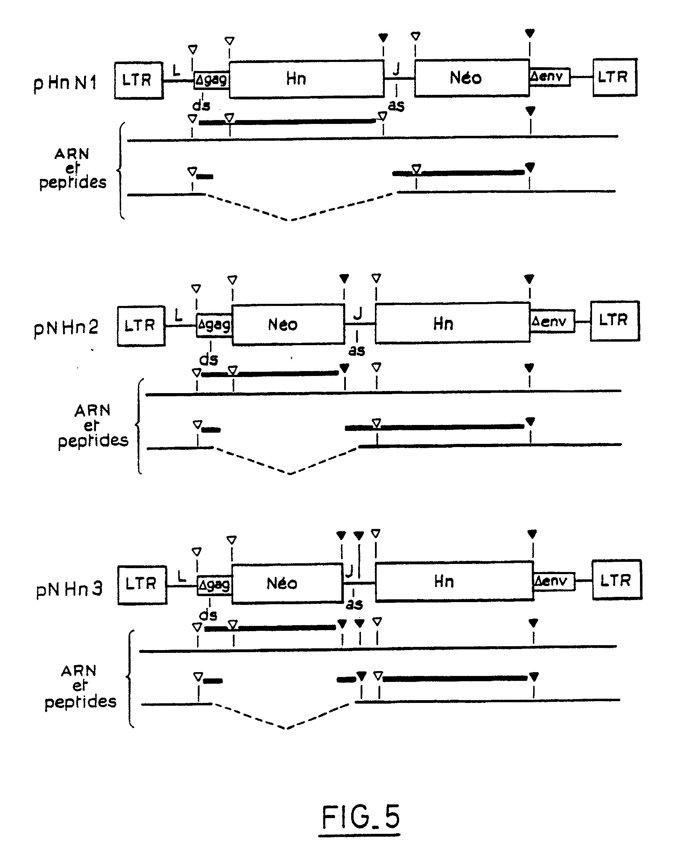

Les structures des vecteurs pNHni1, pNHn2, pNHn3, sont représentées sur la figure 5.

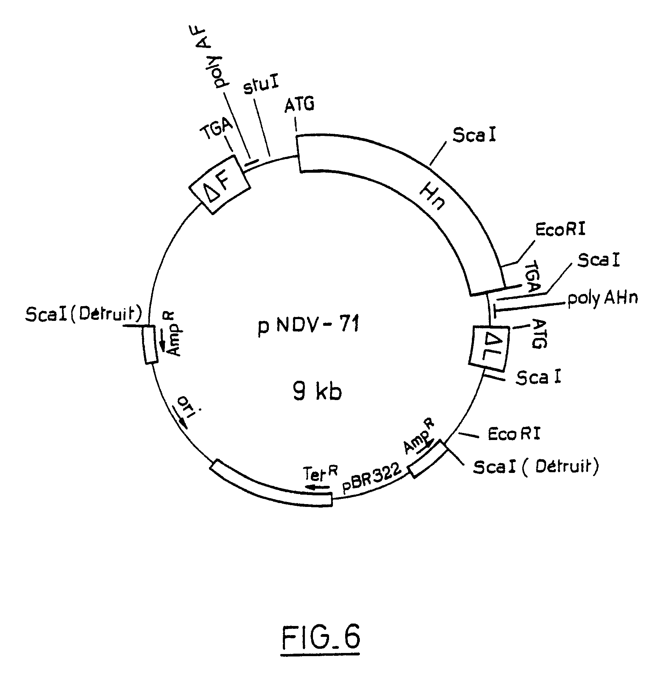

La carte du vecteur PNDV71 est représentée sur la figure 6.

## 2. Production de stocks viraux véhiculant le gène Hn

Les tests en culture cellulaire de ces trois vecteurs (en immunocytochimie, ou en hémadsorption) ont montré que ces vecteurs permettent une expression de la protéine HN. Le vecteur pNHn3 permet une expression authentique de la protéine Hn, (c'est-à-dire sans peptide fusion en 5$^{'}$ qui est donc pleinement fonctionnelle. C'est pourquoi, c'est cette construction qui a été employée pour la suite des travaux : production de stocks viraux, et essais de vaccinations.

La constructions pNHn3 a été transfectée dans la lignée transcomplémentante Isolde. Il a été établi des cultures poyclonales Isolde-NHn3, pouvant produire environ 5x10$^4$ RFU/ml de surnageant. Ces stocks viraux ont été étudiés après infection de cellules de la lignée QT6 ou de CEF (cellules d'embryon de poulets). Dans tous les cas, la propagation du vecteur NHn3 est exempte de celle d'un virus assistant. Sur des colonies cellulaires néo + obtenues après infection par le virus NHn3, on a réalisé des hémadsorptions, et on a pu montrer que 80% de ces clones peuvent agglutiner des érythrocytes.

## 3. Vaccination par l'intermédiaire de cellules présentant l'antigène

### 3.1 Préparation des antigènes associés aux menbranes

Les cellules sont préparées de la façon suivante : des CEFs (ou des cellules de la lignée LMH dans une deuxième expérience) sont infectés par un stock viral helper-free NHn3, sélectionnées par le G418 et amplifiés après apparition de colonies néo +. Les cellules sont alors rincées (par du PBS-Phosphate Buffered Saline-) irradiées par les UV (3 mn, 253 nm) pour détruire le matériel génétique, et récoltées par grattage (ou autres modalités dans le cas de la deuxième expérience). On concentre alors ces cellules par centrigugation, de façon à obtenir environ 10 cellules par ml de PBS. Ces préparations sont alors congelées, afin de lyser les membranes, et ainsi stockées.

### 3.2 Vaccination d'animaux adultes

Deux expériences ont été réalisées. La première visait à montrer ce que l'on pouvait attendre d'un tel procédé : nous sommes donc partis d'une forte quantité de cellules recombinantes (10$^7$ par animal), injectées avec l'adjuvant le plus efficace (Adjuvant Complet de Freud : ACF), et avec un rappel.

La deuxième expérience visait à affiner les résultats positifs du premier protocole, et à les améliorer dans le sens d'une plus grande efficacité de la méthode. Ceci a été fait en testant divers modes de préparations de l'antigène.

**Première expérience**

Des groupes de 10 poulets âgés de 3 semaines ont été immunisés par voie intrapéritonéale, par $10^7$ cellules par animal, en deux injections espacées de 15 jours, et en présence, ou non, d'adjuvant. ENtre les divers groupes, les différences portent sur l'addition d'adjuvant, à savoir son administration lors de chaque injection, ou seulement lors du rappel, ou enfin en absence d'adjuvant.

3 semaines après le rappel, tous les animaux sont injectés intrapéritonéalement par $10^{5,3}$ particules viales du virus de la maladie de Newcastle (NDV souche Texas) comme épreuve virulente.

Les résultats obtenus sont présentés dans le tableau 1. L'efficacité de vaccination est représentée par le nombre d'animaux vivants 7 jours après l'épreuve virulente par le NDV.

La modalité donnant 100% de résistance à une épreuve virulente correspond à celle dans laquelle les cellules recombinantes ont été injectées en présence d'ACF, simultanément lors de l'injection initiale, et lors du rappel. Les deux autres modalités ne sont que partiellement efficaces (10 à 20% d'efficacité). Le groupe 5 est constitué par des animaux témoins ne subissant aucune injection immunisante.

TABLEAU 1

| groupes | effectifs | primo-injection (a) | rappel (a) | nombres sujets sains | % |
|---------|-----------|---------------------|------------|----------------------|---|
| 1 | 10 | $10^7$ CEFR | $10^7$ CEFR + ACF | 1 | 10 |
| 2 | 10 | $10^7$ CEFR | $10^7$ CEFR | 2 | 20 |
| 3 | 9 | $10^7$ CEFR + ACF | $10^7$ CEFR + ACF | 9 | 100 |
| 4 | 10 | $10^7$ CEFT | $10^7$ CEFT + ACF | 0 | 0 |
| 5 | 9 | rien | rien | 0 | 0 |

(a): quantité de cellules injectées. CEF R: CEF infectés par le vecteur NHn3, CEF T: CEF non infectés, ACF : injection en présence de l'adjuvant complet de Freund.
Vaccination anti-NDV: résultats du premier protocole

**Deuxième expérience : améloration de la présentation de l'antigène**

Dans une deuxième expérience, plusieurs conditions d'immunisations ont été comparées (voir tableau 2). Des CEFs infectés par le stock viral NHn3 ont été préparés de manière similaire à la condition employée lors de la première expérience. Ce matériel a été utilisé pour analyser :

1) en présence d'ACF, l'effet d'une injection unique (CEF ACF)

2) l'effet d'un autre type d'adjuvant (adjuvant huileux : AH) en pratiquant une seule injection d'immunisation (CEF AH).

L'influence d'un autre type cellulaire que les CEFs a été étudié. Pour cela, des cellules de la lignée LMH (issues d'un hépatome de poulet induit par voie chimique - Kawaguchi et al., 1987, Cancer Research, 47:4460-4464) ont été infectées par le vecteur NHn3, sélectionnées par le G418, et traitées comme évoqués paragraphe 3.1. Ce matériel a été utilisé pour analyser :

3) l'effet de ces cellules en présence d'adjuvant AH (LMH-AH) lors de simple ou double injection en rappel.

4) divers modes de préparation de ces cellules comme matériel d'immunisation : après action de la trypsine, de la collagénase, après action des ultra-sons ("soniqués") sur les cellules récupérées par grattage, enfin après broyage ("broyés") par ultraturax.

Des contrôles d'efficacité de vaccination ont été effectués comme lors de la première expérience : injection par le virus d'épreuve NDV, 3 semaines après la dernière injection vaccinante par voie intrapéritonéale, et contrôle de la mortalité (après 7 jours) entre les divers groupes immunisés par rapport à des témoins non vaccines.

Les conditions 11, 12 et 13 constituent les contrôles : CEFs (11), ou LMH (12) non infectées par le vecteur NHn3 ; et animaux témoins ne subissant aucune injection (13).

TABLEAU 2

| Groupes | Effectif | TO(primo-injection) | T15 (rappel) | protégés | % protection |
|---|---|---|---|---|---|
| 1 | 9 | $10^7$ CEF/grattage ACF | $10_7$ CEF/grattage ACF | 5 | 55 |
| 2 | 10 | - | $10_7$ CEF/grattage ACF | 0 | 0 |
| 3 | 9 | $10^7$ CEF/grattage ACF | $10_7$ CEF/grattage ACF | 2 | 20 |
| 4 | 10 | - | $10_7$ CEF/grattage AH | 0 | 0 |
| 5 | 10 | - | $10_7$ LMH/trypsine AH | 1 | 10 |
| 6 | 10 | - | $10_7$ LMH/collagénase AH | 0 | 0 |
| 7 | 10 | - | $10_7$ LMH/soniqués AH | 1 | 10 |
| 8 | 10 | - | $10_7$ LMH/broyés AH | 1 | 10 |
| 9 | 10 | - | $10_7$ LMH/grattage AH | 4 | 40 |
| 10 | 10 | - | $10_7$ LMH/grattage AH | 6 | 60 |
| 11 | 10 | $10_7$ CEF témoins AH | - | 0 | 0 |
| 12 | 10 | $10_7$ LMH témoins AH | - | 0 | 0 |
| 13 | 10 | - | - | 0 | 0 |
| ACF : adjuvant complet de Freund, AH : adjuvant huileux. | | | | | |
| Vaccination anti-NDV : deuxième protocole | | | | | |

## 3.3. Conclusion

Des cellules aviaires exprimant à leur surface la protéine HN de la souche Texas du virus de la maladie de Newcastle ont été obtenues par infection à l'aide d'un vecteur rétroviral véhiculant, et induisant l'expression du gène Hn.

Après traitement pour détruire la totalité des acides nucléiques, et briser l'intégralité des cellules, celles-ci injectées à des poulets peuvent, dans certaines conditions, induire une réponse immunitaire et une protection vis à vis d'une épreuve virulente du NDV ($10^{5.3}$ particules virales de la souche Texas).

Les facteurs intervenant sur une plus grande efficacité de la protection semblent être : 1) le type d'adjuvant ; 2) le type cellulaire 3) les traitements susceptibles d'altérer les membranes de la préparation immunisante.

L'adjuvant le plus efficace est l'adjuvant complet de Freud, injecté simultanément lors d'une primo-immunisation, et d'un rappel. D'autres types d'adjuvant pourraient être testés, bien qu'en principe l'adjuvant huileux semble être le plus adapté aux vaccins aviaires.

L'utilisation des cellules de la lignée hépatique LMH présentant de l'antigène HN, par rapport à l'utilisation de fibroblastes embryonnaires (CEF), semble constituer un matériel immunisant plus favorable puisque, d'une part, une seule immunisation par LMH-NHn3 entraîne une protection supérieure à celle obtenue avec des CEF-NHn3, et d'autre part, ces cellules étant transformées et constituant une lignée pérenne, il est alors possible d'amplifier indéfiniment des LMH-NHn3, les congeler, et constituer ainsi une lignée de cellules à caractère immunisant.

La modalité la plus efficace pour obtenir une protection, est représentée par la technique de base : les cellules recombinantes sont récoltées par grattage, soumises à l'action des UV, et directement congelées. Des traitements supplémentaires tels que action d'enzymes protéolytiques, sonication par les ultra-sons, ou broyage fin à l'ultraturax réduisent l'efficacité des préparations.

## Revendications

1. Procédé de préparation d'une composition immunisante constituée par l'association d'une protéine immunogène et de fragments membranaires de cellules, ladite protéine étant produite par lesdites cellules présentée sous forme intégrée à leurs membranes, caractérisé en ce que ladite protéine immunogène est produite par l'expression dans des cellules hôtes appropriées d'un vecteur d'intégration et d'expression d'un gène étranger codant pour ladite protéine dans lesdites cellules hôtes.

2. Procédé selon la revendication 1, caractérisé en ce que ledit vecteur d'intégration est un vecteur rétroviral défectif comportant un gène de sélection et le gène de ladite protéine immunogène, le vecteur étant construit de façon à optimiser la production de ladite protéine et de sorte qu'elle s'intègre à la membrane des cellules dans laquelle elle est produite.

3. Procédé selon la revendication 2, caractérisé en ce que lesdites cellules hôtes sont des cellules aviaires.

4. Procédé selon la revendication 3 caractérisé en ce que lesdites cellules sont des cellules CEF ou LMH de poulets.

5. Procédé selon l'une des revendications 1 à 4 caractérisée en ce que la composition comporte en outre un adjuvant.

6. Procédé selon la revendication 5 caractérisé en ce que l'adjuvant est l'adjuvant complet de Freund.

7. Procédé de préparation d'une composition selon l'une des revendications 1 à 6 caractérisé en ce qu'il comporte les étapes suivantes :

a) la protéine immunogène est produite par l'expression dans des cellules hôtes appropriées d'un vecteur d'intégration et d'expression d'un gène étranger codant pour ladite protéine dans lesdites cellules hôtes, le vecteur étant construit de façon à optimiser la production de ladite protéine et de sorte qu'elle s'intègre à la membrane des cellules dans laquelle elle est produite,

b) les cellules hôtes sont soumises à sélection notamment par l'antibiotique correspondant au gène de sélection, et les cellules ainsi sélectionnées sont multipliées,

c) on traite les cellules obtenues de façon à inactiver les acides nucléiques cellulaires et viraux qu'elles contiennent,

d) on récupère et purifie au besoin les constituants membranaires associés à la protéine immunogène.

8. Procédé selon la revendication 7, caractérisé en ce que ledit vecteur d'intégration est un vecteur rétroviral défectif comportant un gène de sélection et le gène de ladite protéine immunogène.

9. Procédé de préparation d'une composition immunisante selon l'une des revendication précédentes, caractérisé en ce que :

a) on transfecte un vecteur rétroviral défectif comportant un gène de sélection et le gène de la protéine immunogène sur une culture de cellules assistantes ("helper") présenté sous forme d'ADN plasmidien. Il en résulte la production de ce même vecteur sous forme de virus défectif doué de capacités infectieuses,

b) on infecte, avec la préparation virale helper-free ainsi obtenue, une culture desdites cellules hôtes, puis

c) les cellules hôtes sont soumises à sélection notamment par l'antibiotique correspondant au gène de sélection, et les cellules ainsi sélectionnées sont multipliées,

d) on traite les cellules obtenues de façon à inactiver les acides nucléiques cellulaires et viraux qu'elles contiennent,

e) on récupère et purifie au besoin les constituants membranaires associés à la protéine immunogène

10. Procédé selon la revendication précédente, caractérisé en ce que les vecteurs viraux, les cellules hôtes et les cellules assistantes le cas échéant, sont d'origine aviaire.

11. Procédé selon les revendication précédentes, caractérisé en ce que le vecteur viral d'intégration et d'expression du gène de la protéine immunogène dans des cellules aviaires est constitué par tout ou partie du génome proviral de l'érythroblastose aviaire ou d'un virus apparenté dans lequel les gènes hétérologues à savoir le gène de sélection et le gène de la protéine immunogène remplacent les gènes v-erbA et v-erbB et en ce que lesdits gènes se trouvent soit sous le contrôle d'un promoteur de LTR du même virus auquel cas les gènes hétérologues miment les gènes qu'ils remplacent soit sous le contrôle d'un promoteur hétérologue auquel cas une séquence att additionnelle est positionnée en amont dudit promoteur hétérologue.

12. Procédé selon la revendication précédente, caractérisé en ce que le vecteur viral est utilisé pour une intégration dans des cellules de poulet telles que des cellules CEF ou LMH et les gènes hétérologues se trouvent sous la dépendance d'un même promoteur de LTR aviaire et miment les gènes qu'ils remplacent.

13. Procédé selon la revendication précédente, caractérisé en ce que le vecteur viral d'intégration, le gène de sélection est situé en position v-erbA et le gène correspondant à la protéine immunogène est situé en position v-erbB.

14. Procédé selon l'une des revendications précédentes, caractérisé en ce que les gènes hétérologues sont traduits à partir de l'AUG du gène gag ou à partir de leur propre AUG mais toujours dans le même cadre de lecture que celui du gène gag.

15. Procédé selon l'une des revendications précédentes, caractérisé en ce que le gène de sélection est traduit à partir de l'AUG du gène gag et le gène de la protéine immunogène est traduit à partir de l'AUG du gène gag ou son propre AUG.

16. Procédé selon l'une des revendications précédentes, caractérisé en ce qu'un codon stop est introduit entre le gène gag et le gène de la protéine immunogène ou l'AUG propre dudit gène de la protéine immunogène.

17. Procédé selon l'une des revendications précédentes, caractérisé en ce que les cellules helper sont des cellules de cailles QT6 obtenues à l'aide d'un vecteur capable de transformer une cellule QT6 normal en cellule assistante comportant tout ou partie des trois gènes gag-pol-env du virus RAV- 1 ou RAV-2, placés sous le contrôle transcriptionnel d'une LTR du même virus à laquelle diverses délétions ont été apportées afin de supprimer l'aptitude à l'encapsidation de l'ARN produit par ce vecteur.

18. Procédé d'une composition immunisante contre le virus RSV selon l'une des revendications précédentes, caractérisé en ce que ladite protéine est la protéine env.

19. Procédé de préparation d'une composition immunisante contre la maladie de Newcastle selon l'une des revendications 1 à 17 caractérisé en ce que ladite protéine est l'hémaglutinine-neuraminidase (HN) du virus de la maladie de Newcastle.

20. Composition immunisante obtenue par le procédé d'une des revendications 1 à 20.

FIG. 1

FIG. 2

FIG.3

FIG_4

FIG.5

FIG. 6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | WO-A-8 903 877 (INSTITUT NATIONAL DE LA RECHERCHE AGRONOMIQUE (INRA)) <br> * revendications * <br> — — — | 1-20 | C 12 N 15/86 <br> C 12 N 15/45 <br> C 12 N 15/48 <br> A 61 K 39/12 <br> A 61 K 39/17 <br> A 61 K 39/21 |
| Y | VIROLOGY vol. 171, no. 1, juillet 1989, New York, Etats-Unis pages 10 - 17; T. MORRISON et al.: "Avian cells expressing the Newcastle Disease Virus hemagglutinin neuraminidase protein are resistant to Newcastle Disease Virus infection" <br> * abrégé * <br> — — — | 1-20 | |
| A | INT. J. CANCER vol. 25, no. 3, mars 1980, Genève, Suisse pages 355 - 362; M. PRAT et al.: "Interaction between cellular and viral genes in the expression of the RSV-induced transformation-specific cell-surface antigen VCSA" <br> * abrégé * <br> — — — — — | 1-20 | |

|  | DOMAINES TECHNIQUES RECHERCHES (Int. Cl.5) |
|---|---|
|  | A 61 K <br> C 07 K <br> C 12 N |

Le présent rapport de recherche a été établi pour toutes les revendications

| Lieu de la recherche | Date d'achèvement de la recherche | Examinateur |
|---|---|---|
| La Haye | 16 janvier 91 | NOOIJ F.J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

 

& : membre de la même famille, document correspondant